# EUROPÄISCHE PATENTSCHRIFT

(11) **EP 1 639 047 B1**
(45) Veröffentlichungstag und Bekanntmachung des Hinweises auf die Patenterteilung: **23.04.2008**
(21) Anmeldenummer: 04739281.6
(22) Anmeldetag: 21.05.2004
(51) Int. Cl.: C09B 57/04, C09B 17/00

(54) **HETEROCYCLISCHE FARBMITTEL AUF BASIS VON DIAZABENZOISOINDOLEN**
HETEROCYCLIC COLORANTS BASED ON DIAZABENZOISOINDOLES
COLORANTS HETEROCYCLIQUES A BASE DE DIAZABENZO-ISO-INDOLS

(30) Priorität: 11.06.2003 DE 10326211
(43) Veröffentlichungstag der Anmeldung: 29.03.2006
(73) Patentinhaber: Clariant Produkte (Deutschland) GmbH, 65929 Frankfurt am Main (DE)
(72) Erfinder: HECKMANN, Heino, 65835 Liederbach (DE); METZ, Hans, Joachim, 64285 Darmstadt (DE)
(86) Internationale Anmeldenummer: PCT/EP2004/005459
(87) Internationale Veröffentlichungsnummer: WO 2004/108836

(56) Entgegenhaltungen:
- EP-A- 0 038 548
- DE-A- 19 644 077
- US-A- 4 166 179
- US-A- 4 384 060

## Beschreibung

Die vorliegende Erfindung betrifft neue organische Farbmittel.

Auf dem Gebiet der Farbpigmente besteht eine ständige Marktnachfrage nach neuen Farbnuancen, die hohe Migrations-, Licht und Lösungsmittelechtheiten, gute Wärmestabilitäten und eine hohe Färbekraft aufweisen.

In US 4,166,179 werden unter anderem Pigmente beansprucht, die sich von 4,7-Diazaisoindol ableiten, eine zusätzliche Benzannellierung am Indolgerüst ist dabei jedoch nicht beschrieben.

Es bestand die Aufgabe, neue Farbmittel zum Einfärben oder Pigmentieren von organischen oder anorganischen, hoch- oder niedermolekularen, insbesondere hochmolekularen organischen Materialien zu finden, die von einfach zugänglichen Zwischenprodukten ausgehen.

Es wurde gefunden, dass diese Aufgabe überraschenderweise durch Verbindungen der Formel (I) gelöst wird.

Ein Gegenstand der vorliegenden Erfindung sind somit Verbindungen der allgemeinen Formel (I) 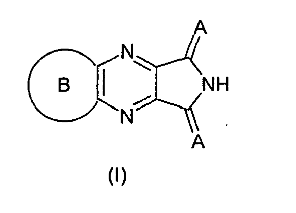 worin A eine Gruppe der allgemeinen Formel (II), (III), (IV) oder (V) ist 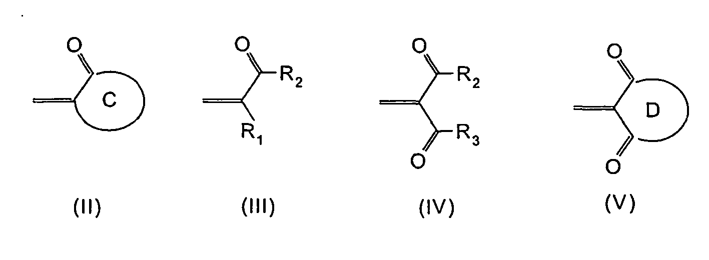 worin C und D für eine alicyclische oder heterocyclische Gruppe stehen;
R₁ für CN oder für einen 5- bis 7-gliedrigen heteroaromatischen Rest mit 1, 2 oder 3 Heteroatomen aus der Gruppe N, O und S steht,
und R₂ und R₃ unabhängig voneinander C₁-C₂₅-Alkyl, C₅-C₁₂-Cycloalkyl, C₆-C₂₄-Aryl, OH, OR₄ oder NR₄R₅ bedeuten, worin R₄ und R₅ gleich oder verschieden sind und für Wasserstoff, C₁-C₂₅-Alkyl, C₅-C₁₂-Cycloalkyl, unsubstituiertes oder durch 1, 2, 3 oder 4 Reste Halogen, R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, SO₂NHR⁰, SO₂NR⁰₂ oder durch einen 5- bis 7-gliedrigen heteroaromatischen Rest mit 1, 2 oder 3 Heteroatomen aus der Gruppe N, O und S substituiertes C₆-C₂₄-Aryl oder einen 5- bis 7-gliedrigen heteroaromatischen Rest mit 1, 2 oder 3 Heteroatomen aus der Gruppe N, O und S stehen,
wobei R⁰ für C₁-C₁₈-Alkyl oder C₆-C₂₄-Aryl steht;
und B unsubstituiertes oder 1- bis 4-fach substituiertes ortho-C₆-C₁₈-Arylen bedeutet,
wobei als Substituenten vorzugsweise Halogene, R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, SO₂NHR⁰, SO₂NR⁰₂ oder ein 5- bis 7-gliedriger heteroaromatischer Rest mit 1, 2 oder 3 Heteroatomen aus der Gruppe N, O und S in Betracht kommen.

Bevorzugt sind weiterhin Verbindungen der Formel (I), worin B ortho-Phenylen oder 2,3-Naphthylen ist.

Besonders bevorzugt sind die Verbindungen der Formel (I), worin A einen zweiwertigen alicyclischen oder heterocyclischen Rest der Formeln (a) bis (g) bedeutet, 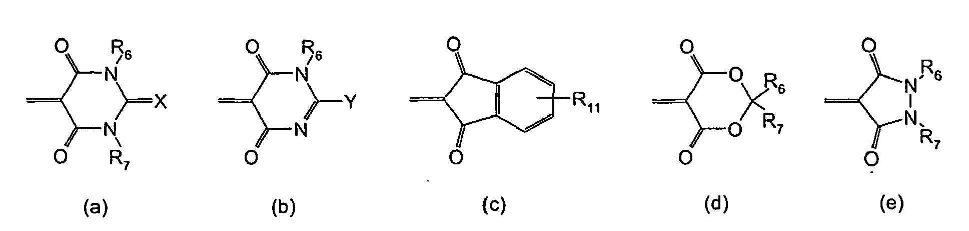 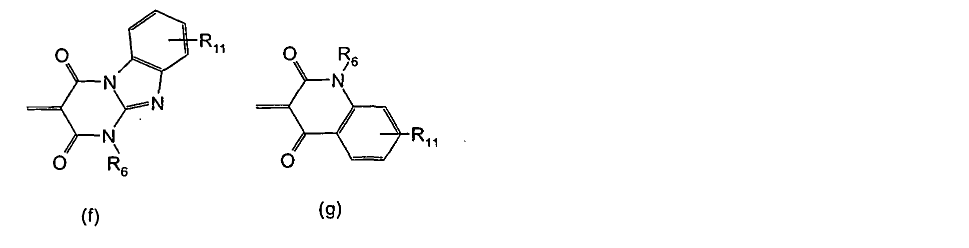 wobei R₆ und R₇ unabhängig voneinander für Wasserstoff, C₁-C₂₅-Alkyl, C₅-C₁₂-Cycloalkyl, C₆-C₂₄-Aryl, C₁-C₂₅-Alkyl-(C₆-C₁₀-aryl), einen 5- bis 7-gliedrigen heteroaromatischen Rest mit 1, 2 oder 3 Heteroatomen aus der Gruppe N, O und S, -(CH₂)ₙ-COR₈ oder -(CH₂)ₘ-OR₉, stehen, worin R₈ für Hydroxy, Amino, unsubstituiertes oder ein- oder mehrfach mit Hydroxy oder Amino substituiertes C₁-C₂₅-Alkoxy, C₁-C₂₅-Alkylamino, Di-(C₁-C₂₅-alkyl)-amino,
C₁-C₂₅-Alkyl-(C₆-C₁₀-aryl)-amino, (C₆-C₂₄-Aryl)-amino, Di-(C₆-C₂₄-Aryl)-amino, C₁-C₂₅-Alkyl-(C₆-C₁₀-aryl)-amino, oder C₂-C₂₄-Alkenyloxy steht,
und R₉ für Wasserstoff oder -CO-(C₁-C₂₅-Alkyl) steht, und n und m unabhängig voneinander für eine ganze Zahl von 0 bis 6, bevorzugt 1 bis 4, stehen, und worin in R₆, R₇, R₈ und R₉ eine C-C-Einheit auch durch eine Ethereinheit C-O-C ersetzt sein kann,
X für =O, =S oder =NR₁₀ steht, worin R₁₀ eine der Bedeutungen von R₆ hat,
Y für Wasserstoff, R₇, OR₇, SR₇, NHCN oder NR₇R₁₀ steht,
und R₁₁ Wasserstoff, Halogen, CN, R₇, OR₇, SR₇, NR₇R₁₀, NO₂, SO₂(OR₇), SO₂R₇, SO₂NHR₇, SO₂N(R₇)₂ oder PO₂(OR₇) bedeutet.

R₆ und R₇ sind besonders bevorzugt Wasserstoff, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, Hydroxycarbonyl-C₀-C₆-alkyl, C₁-C₁₈-Alkoxycarbonyl-C₀-C₆-alkyl, Aminocarbonyl-C₀-C₆-alkyl, C₁-C₁₈-Alkylaminocarbonyl-C₀-C₆-alkyl, C₆-C₁₀-Arylaminocarbonyl-C₀-C₆-alkyl, Di(C₁-C₁₈-alkyl)-aminocarbonyl-C₀-C₆-alkyl, C₁-C₁₈-Alkyl-C₆-C₁₀-arylaminocarbonyl-C₀-C₆-alkyl und Di(C₆-C₁₀-aryl)-aminocarbonyl-C₀-C₆-alkyl.

R₈ ist besonders bevorzugt Hydroxy, C₁-C₁₈-Alkoxy, C₁-C₁₈-Alkylamino, Di(C₁-C₁₈-alkyl)-amino, Benzylamino, C₆-C₁₀-Arylamino, Di(C₆-C₁₀-aryl)-amino oder (C₂-C₁₈)-Alkenyloxy.

R₁₁ ist besonders bevorzugt Wasserstoff, Cl, Br, C₁-C₁₈-Alkyl, C₅-C₆-Cycloalkyl, Benzyl, C₆-C₁₀-Aryl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈-Alkylthio, C₆-C₁₀-Arylthio, C₁-C₁₈-Alkylamino, C₆-C₁₀-Arylamino, Di(C₁-C₁₈-alkyl)-amino, C₁-C₁₈-Alkyl-(C₆-C₁₀-aryl)-amino, Di(C₆-C₁₀-aryl)-amino, SO₃H, C₁-C₁₈-Alkoxysulfonyl, C₁-C₁₈-Alkylsulfonyl, C₁-C₁₈-alkylaminosulfonyl und Di(C₁-C₁₈-alkyl)-aminosulfonyl.

Ein weiterer Gegenstand der vorliegenden Erfindung ist ein Verfahren zur Herstellung von Verbindungen der allgemeinen Formel (I) durch Umsetzung eines 2,3-Dicyanochinoxalins der Formel (XIV) 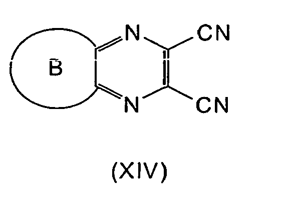 mit insgesamt mindestens 2 Equivalenten Ammoniak und/oder Alkoholaten MOR₁₂, worin M Natrium oder Kalium bedeutet, wie z.B. Natriummethylat, Natriumethylat, Natriumamylat, Kaliummethylat oder Kalium-*tert*.-butylat, zu di-oder monoiminosubstituierten Diazabenzoisoindolen, die 0 bis 2 Alkoxysubstituenten tragen, beispielhaft repräsentiert durch Verbindungen der Formeln (VI), (VII) oder (VIII), 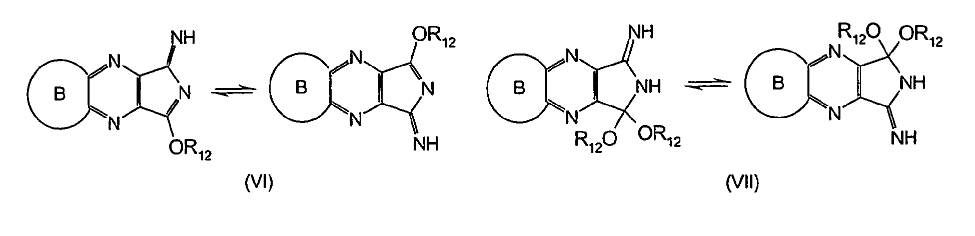 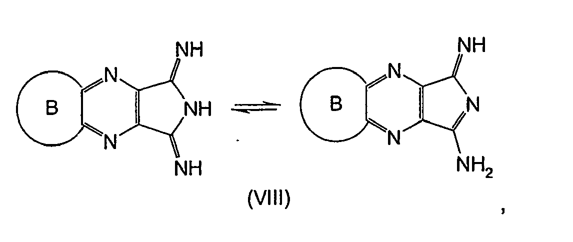 worin R₁₂ für C₁-C₁₈-Alkyl oder -(CH₂)ₘ-OH steht und m eine ganze Zahl im Bereich von 1 bis 6 bedeutet, und eine C-C- Einheit auch durch eine Ethereinheit C-O-C ersetzt werden kann,
in einem Lösemittel oder Lösemittelgemisch unter basischen bis neutralen Bedingungen bei einer Temperatur von -20 bis 120°C, bevorzugt 0 bis 100°C, besonders bevorzugt 20 bis 80°C,
die anschließend nach Zwischenisolierung oder ohne zwischenisoliert zu werden in einem Lösungsmittel oder Lösungsmittelgemisch unter neutralen bis sauren Bedingungen, vorzugsweise in Gegenwart einer organischen Säure, wie z.B. Ameisensäure, Essigsäure oder Propionsäure, oder einer anorganischen Säure, wie z.B. Schwefelsäure, Salzsäure oder Phosphorsäure, und zweckmäßigerweise bei einer Temperatur von 10 bis 250°C, insbesondere 20 bis 200°C, besonders bevorzugt 30 bis 150°C, mit mindestens 2 Equivalenten einer Verbindung der Formeln (IX), (X), (XI) oder (XII) 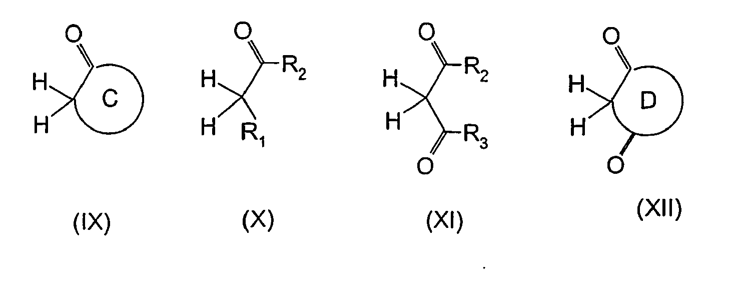 worin C, D, R₁, R₂ und R₃ wie vorstehend definiert sind,
zu einem weiteren Zwischenprodukt der allgemeinen Formel (XIIIa) oder (XIIIb) umgesetzt werden 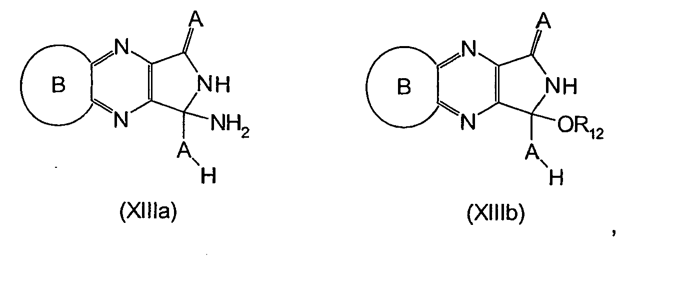 von dem anschließend ein Mol Ammoniak oder HOR₁₂ eliminiert wird.

Die Eliminierung von Ammoniak und/oder HOR₁₂ zu den erfindungsgemässen Verbindungen der Formel (I) erfolgt zweckmäßigerweise bei einer Temperatur von -30 bis 250°C, bevorzugt -20 bis 200°C, besonders bevorzugt 0 bis 150°C, vorzugsweise in Gegenwart eines Lösemittels wie Wasser, Methanol, Ethanol, Glykolen, Dimethylformamid, N-Methylpyrrolidon, Toluol, sowie besonders bevorzugt in Gegenwart einer organischen Säure, wie z.B. Ameisensäure, Essigsäure oder Propionsäure, oder in Gegenwart einer anorganischen Säure, wie z.B. Schwefelsäure, Salzsäure oder Phosphorsäure.

2,3-Dicyanochinoxaline der Formel (XIV) können gemäß Liebigs Ann. Chem. 1981, 2, Seiten 333-341, durch Umsetzung von entsprechenden 2,3-Dichlorchinoxalinen der Formel (XV) 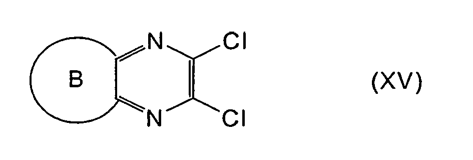 mit mindestens der zweifachen molaren Menge Tetraethylammoniumcyanid in DMSO hergestellt werden.
Überraschenderweise wurde nun gefunden, dass auch ein Cyanid eines Hauptgruppen- oder Übergangsgruppenmetalls in einem organischen Lösungsmittel in Gegenwart eines Phasentransferkatalysators bei erhöhten Temperaturen eingesetzt werden kann. Bevorzugt sind Cyanide eines Alkali- oder Erdalkalimetalls, CuCN, Zn(CN)₂ oder Fe(CN)₂, vorzugsweise Natriumcyanid. Vorzugsweise geschieht die Umsetzung von Verbindungen der allgemeinen Formel (XV) in einem organischen Lösungsmittel, das eine hohe Dielektrizitätskonstante aufweist, wie zum Beispiel DMSO, DMF, Dimethylacetamid, NMP, Acetonitril, Sulfolan und Dioxolan, bevorzugt DMSO und NMP, insbesondere DMSO, in Gegenwart eines Phasentransferkatalysators, ausgewählt aus der Gruppe der quarternären Alkylammoniumsalze, insbesondere quarternäre Alkylammoniumhalogenide, wie beispielsweise Distearyldimethylammoniumchlorid, Trimethylbenzylammoniumbromid oder Tetrabutylammoniumchlorid, bei Temperaturen von 0 bis 150°C, insbesondere bei 10 bis 100°C, vorzugsweise bei 20 bis 80°C.
Der Vorteil des neuen Verfahrens liegt darin, dass von dem preisgünstigeren und im Gegensatz zum organischen Cyanid weniger hygroskopischen Natriumcyanid, das zudem eine deutlich geringere Toxizität aufweist, ausgegangen werden kann, und dass der Phasentransferkatalysator in unterstöchiometrischer molarer Menge, bevorzugt in 0,01 bis 0,9 fache molarer Menge, bezogen auf das Ausgangsprodukt der Formel (XV), eingesetzt werden kann.

Die vorliegende Erfindung betrifft des weiteren ein Zwischenprodukt der allgemeinen Formel (XIIIa),

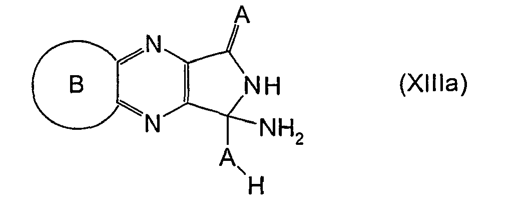

Erfindungsgemäße Verbindungen der allgemeinen Formel (I) werden zum Einfärben oder Pigmentieren von organischen oder anorganischen, hoch- oder niedermolekularen, insbesondere hochmolekularen organischen Materialien verwendet.

Je nach Art ihrer Substituenten und des zu färbenden hochmolekularen organischen Materials können die erfindungsgemäßen Verbindungen als polymerlösliche Farbstoffe oder als Pigmente verwendet werden. Im letzteren Fall ist es vorteilhaft, die bei der Synthese anfallenden Produkte (Rohpigmente) durch Nachbehandlung in organischen Lösungsmitteln, in denen die Pigmente selbst nicht gelöst werden, und bei erhöhten Temperaturen, beispielsweise bei 60 bis 200°C, insbesondere bei 70 bis 150°C, vorzugsweise bei 75 bis 100°C, in eine feindisperse Form mit oft weiter verbesserten Pigmenteigenschaften zu überführen. Die Nachbehandlung wird vorzugsweise mit einer Mahl- oder Knetoperation kombiniert.

Die erfindungsgemäßen Farbmittel eignen sich ausgezeichnet zum Färben von hochmolekularen Materialien, die organischer oder anorganischer Natur sein können, und Kunststoffe und/oder Naturstoffe bedeuten. Es kann sich zum Beispiel um Naturharze, trocknende Öle, Kautschuk oder Casein handeln. Es kann sich aber auch um abgewandelte Naturstoffe handeln, wie beispielsweise Chlorkautschuk, ölmodifizierte Alkydharze, Viskose, Cellulosederivate, wie Celluloseester oder Celluloseether, und insbesondere um vollsynthetische organische Polymere (Kunststoffe), die durch Polymerisation, Polykondensation oder Polyaddition erhalten werden können. Aus der Klasse der durch Polymerisation hergestellten Kunststoffe seien besonders folgende genannt: Polyolefine, wie zum Beispiel Polyethylen, Polypropylen, Polyisobutylen, und substituierte Polyolefine, wie beispielsweise Polystyrol, Polyvinylchlorid, Polyvinylidenchlorid, Polyvinylacetale, Polyacrylnitril, Polyacrylsäure, Polymethacrylsäure, Polyacrylsäure- und Polymethacrylsäureester oder Polybutadien, sowie Copolymerisate davon. Aus der Klasse der durch Polyaddition und Polykondensation hergestellten Kunststoffe seien genannt: Polyester, Polyamide, Polyimide, Polycarbonate, Polyurethane, Polyether, Polyacetale, sowie die Kondensationsprodukte von Formaldehyd mit Phenolen (Phenoplaste) und die Kondensationsprodukte von Formaldehyd mit Harnstoff, Thioharnstoff und Melamin (Aminoplaste). Weiterhin kann es sich auch um Silikone oder Silikonharze handeln.

Solche hochmolekularen Materialien können einzeln oder in Gemischen als plastische Massen, Schmelzen oder in Form von Spinnlösungen vorliegen. Sie können auch in Form ihrer Monomeren oder im polymerisierten Zustand in gelöster Form als Filmbildner oder Bindemittel für Lacke oder Druckfarben vorliegen, wie Leinölfirnis, Nitrocellulose, Alkydharze, Melaminharze und Formaldehydharze oder Acrylharze.

Die erfindungsgemäßen Verbindungen eignen sich demzufolge als Farbmittel in Anstrichfarben auf öliger oder wässriger Grundlage, in Lacken verschiedener Art, Tarnfarben, zum Spinnfärben, zum Massefärben oder Pigmentieren von Kunststoffen, in Druckfarben für das graphische Gewerbe, wie zum Beispiel im Papier-, Textil- oder Dekorationsdruck, und in der Papiermassefärbung, zur Herstellung von Tinten, Ink-Jet Tinten auf wässriger oder nichtwässriger Basis, Mikroemulsionstinten und Tinten, die nach dem Hot-melt Verfahren arbeiten.

Die erfindungsgemäßen Verbindungen sind auch geeignet als Farbmittel in elektrophotographischen Tonern und Entwicklern, wie z.B. Ein- oder Zweikomponentenpulvertonern (auch Ein- oder Zweikomponenten-Entwickler genannt), Magnettoner, Flüssigtoner, Latextoner, Polymerisationstoner sowie Spezialtoner.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Desweiteren sind die erfindungsgemäßen Verbindungen geeignet als Farbmittel in Pulvern und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen.

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Verbindungen als Farbmittel in Tinten, vorzugsweise Ink-Jet Tinten, wie z.B. auf wäßriger oder nichtwäßriger Basis, Mikroemulsionstinten sowie in solchen Tinten, die nach dem hot-melt-Verfahren arbeiten, geeignet.
Ink-Jet-Tinten enthalten im allgemeinen insgesamt 0,5 bis 15 Gew.-%, vorzugsweise 1,5 bis 8 Gew.-%, (trocken gerechnet) einer oder mehrerer der erfindungsgemäßen Verbindungen.
Mikroemulsionstinten basieren auf organischen Lösemitteln, Wasser und ggf. einer zusätzlichen hydrotropen Substanz (Grenzflächenvermittler). Mikroemulsionstinten enthalten 0,5 bis 15 Gew.-%, vorzugsweise 1,5 bis 8 Gew.-%, einer oder mehrerer der erfindungsgemäßen Verbindungen, 5 bis 99 Gew.-% Wasser und 0,5 bis 94,5 Gew.-% organisches Lösungsmittel und/oder hydrotrope Verbindung.

"Solvent based" Ink-Jet-Tinten enthalten vorzugsweise 0,5 bis 15 Gew.-% einer oder mehrerer erfindungsgemäßer Verbindungen, 85 bis 99,5 Gew.-% organisches Lösungsmittel und/oder hydrotrope Verbindungen.

Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60°C und ca. 140°C liegt. Hot-Melt Ink-Jet-Tinten bestehen z.B. im wesentlichen aus 20 bis 90 Gew.-% Wachs und 1 bis 10 Gew.-% einer oder mehrerer der erfindungsgemäßen Verbindungen. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein.

Weiterhin sind die erfindungsgemäßen Farbmittel auch für Farbfilter, sowohl für die additive wie auch für die subtraktive Farberzeugung, sowie als Farbmittel für elektronische Tinten ("electronic inks" bzw. "e-inks") oder "electronic paper" ("e-paper") geeignet.

Bei der Herstellung sogenannter Farbfilter, sowohl reflektierender wie durchsichtiger Farbfilter, werden Pigmente in Form einer Paste oder als pigmentierte Photoresists in geeigneten Bindemitteln (Acrylate, Acrylester, Polyimide, Polyvinylalkohole, Epoxide, Polyester, Melamine, Gelantine, Caseine) auf die jeweiligen LCD-Bauteilen (z.B. TFT-LCD= Thin Film Transistor Liquid Crystal Displays oder z.B. ((S) TN-LCD = (Super) Twisted Nematic-LCD) aufgebracht. Neben einer hohen Thermostabilität ist für eine stabile Paste bzw. einem pigmentierten Photoresist auch eine hohe Pigmentreinheit Voraussetzung.

Darüber hinaus können die pigmentierten Color Filter auch durch Ink Jet-Druckverfahren oder andere geeignete Druckverfahren aufgebracht werden.

Die vorliegende Erfindung betrifft überdies die Verwendung der erfindungsgemäßen Farbmittel in optischen Schichten für die optische Datenspeicherung, bevorzugt für die optische Datenspeicherung, bei der ein Laser zum Schreiben der Daten verwendet wird. Die für diese Anwendung notwendige Löslichkeit der Farbmittel im Anwendungsmedium kann durch die Art und Anzahl der Substituenten eingestellt werden.

Des weiteren eignen sich die erfindungsgemäßen Verbindungen als Farbmittel in Kosmetik, zur Einfärbung von Saatgut und zur Einfärbung von Mineralölen, Schmierfetten und Wachsen.

Je nach Art der Substituenten der erfindungsgemäßen Verbindungen, zeichnen sich die erhaltenen Färbungen durch gute Hitze-, Licht- und Wetterechtheit, Chemikalienbeständigkeit und die sehr guten applikatorischen Eigenschaften, z.B. Kristallisierechtheit und Dispergierechtheit und insbesondere durch ihre Migrier-, Ausblüh-, Überlackier- und Lösungsmittelechtheit aus. Die als polymerlösliche Farbstoffe eingesetzten Verbindungen weisen naturgemäß nur eine geringe oder eingeschränkte Lösungsmittelechtheit auf.

Einen weiteren Erfindungsgegenstand bildet eine Zusammensetzung, enthaltend ein organisches oder anorganisches, hochmolekulares oder niedermolekulares, insbesondere hochmolekulares organisches Material, und mindestens eine erfindungsgemäße Verbindung in einer färberisch wirksamen Menge, in der Regel im Bereich von 0,005 bis 70 Gew.-%, insbesondere von 0,01 bis 10 Gew.-%, bezogen auf das organische oder anorganische Material.

### Beispiele

### Zwischenprodukte

### Beispiel A: 2,3-Dicyanochinoxalin

Ein Gemisch aus 10,0 g 2,3-Dichlorchinoxalin, 5,40 g Natriumcyanid und 9,32 g Benzyltrimethylammoniumchlorid wird in 500 ml DMSO 17 Stunden bei Raumtemperatur gerührt. Unter intensiver Rührung wird das Reaktionsgemisch auf 1000 ml Eiswasser gegossen, 1 Stunde gerührt, abgesaugt und mit Wasser gewaschen. Nach Trocknen bei 60°C werden 8,03 g (89 % d.Th.) hellgrauer Kristalle folgender Formel erhalten 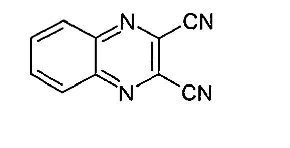 MS (m/e): 181 [M+H]⁺, 203 [M+Na]⁺
H-NMR (DMSO): 8.34 (m, 2H), 8.23 (m, 2H)

### Beispiel B: 2,3-Dicyano-6-methoxychinoxalin

Ein Gemisch aus 31,1 g 2,3-Dichlor-6-methoxychinoxalin, 14,7 g Natriumcyanid und 2,79 g Benzyltrimethylammoniumchlorid wird in 210 ml DMSO 48 Stunden bei Raumtemperatur gerührt. Unter intensiver Rührung wird das Reaktionsgemisch auf 520 ml Eiswasser gegossen, 1 Stunde gerührt, abgesaugt und mit Wasser gewaschen. Nach Trocknen bei 40°C werden 23,8 g (83 % d.Th.) hellgrauer Kristalle folgender Formel erhalten 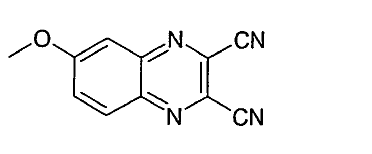 MS (m/e): 211 [M+H]⁺, 233 [M+Na]⁺
H-NMR (DMSO): 8.21 (d, 1 H), 7.83 (dd, 1 H), 7,68 (d, 1 H), 4.02 (s, 3H)

### Beispiel C: 6-Chlor-2,3-dicyano-chinoxalin

Ein Gemisch aus 11,7 g 2,3,6-Trichlorchinoxalin, 5,39 g Natriumcyanid und 2,04 g Benzyltrimethylammoniumchlorid wird in 200 ml DMSO 24 Stunden bei Raumtemperatur gerührt. Unter intensiver Rührung wird das Reaktionsgemisch auf 520 ml Eiswasser gegossen, 1 Stunde gerührt, abgesaugt und mit Wasser gewaschen. Nach Trocknen bei 40°C werden 8,12 g (76 % d.Th.) eines grauen Pulvers einer Verbindung folgender Formel erhalten 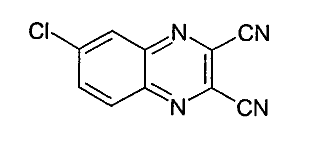 MS (m/e): 215 [M+H]⁺, 237 [M+Na]⁺
H-NMR (DMSO): 8.53 (d, 1 H), 8.37 (d, 1 H), 8.26 (dd, 1 H)

### Beispiel D: 1-Amino-1-(2,4,6-trioxo-tetrahydropyrimidin-5-yl)-3-(2,4,6-trioxotetrahydropyrimidin-5-yliden)-4,9-diazabenzo[f]isoindol

Zu einer Suspension von 175 g 2,3-Dicyanochinoxalin in 1700 ml Methanol werden bei 10°C 23,0 g Natriummethylatlösung (30 % in Methanol) getropft und 16 Stunden bei Raumtemperatur gerührt. Die Mischung wird mit 3500 ml Methanol verdünnt und nach Zugabe von 52,2 g Eisessig und 261 g Barbitursäure 6 Stunden am Rückfluss gerührt. Die Suspension wird nach Abkühlen auf Raumtemperatur filtriert, mit Methanol, dann Wasser gewaschen und bei 60°C getrocknet. Es werden 154 g (84 % d.Th.) eines hellbraunen Pulvers der folgenden Formel erhalten 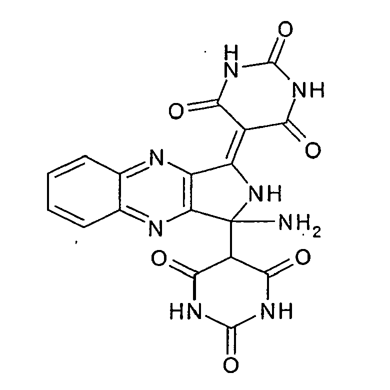 Schmelzpunkt: >300°C
MS (m/e): 437 [M+H]⁺, 459 [M+Na]⁺

### Beispiel E: 1-Amino-1-(1,3-dimethyl-2,4,6-trioxo-tetrahydropyrimidin-5-yl)-3-(1,3-dimethyl-2,4,6-trioxo-tetrahydropyrimidin-5-yliden)-4,9-diazabenzo[f]isoindol

Zu einer Suspension von 22,8 g 2,3-Dicyanochinoxalin in 180 ml Methanol werden bei 10°C 3,0 g Natriummethylatlösung (30 % in Methanol) getropft und 16h bei Raumtemperatur gerührt. Die Mischung wird mit 200 ml Methanol verdünnt und nach Zugabe von 6,8 g Eisessig und 41,6 g 1,3-Dimethylbarbitursäure 6 Stunden am Rückfluss gerührt. Die Suspension wird nach Abkühlen auf Raumtemperatur filtriert, mit Methanol, dann Wasser gewaschen und bei 60°C getrocknet. Es werden 33,3 g (54 % d.Th.) eines beige-braunen Pulvers der folgenden Formel erhalten 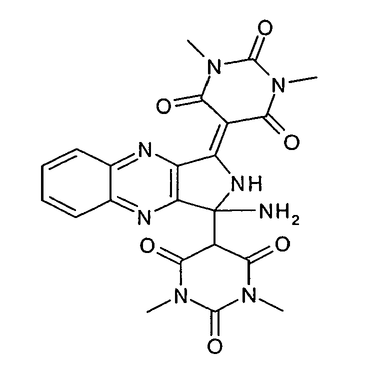 Schmelzpunkt: >300°C
MS (m/e): 493 [M+H]⁺, 515 [M+Na]⁺

### Beispiel F: 1-Amino-1-(1,3-dioxoindan-2-yl)-3-(1,3-dioxoindan-2-yliden)-4,9-diazabenzo[f]isoindol

Zu einer Suspension von 17,5 g 2,3-Dicyanochinoxalin in 170 ml Methanol werden bei 10°C 2,3 g Natriummethylatlösung (30 % in Methanol) getropft und 16 Stunden bei Raumtemperatur gerührt. Die Mischung wird mit 360 ml Methanol verdünnt und nach Zugabe von 5,2 g Eisessig und 29,8 g 1,3-Dioxoindan 6 Stunden am Rückfluss gerührt. Die Suspension wird nach Abkühlen auf Raumtemperatur filtriert, mit Methanol, dann Wasser gewaschen und bei 60°C getrocknet. Es werden 31,5 g (69 % d.Th.) eines braunen Pulvers der folgenden Formel erhalten 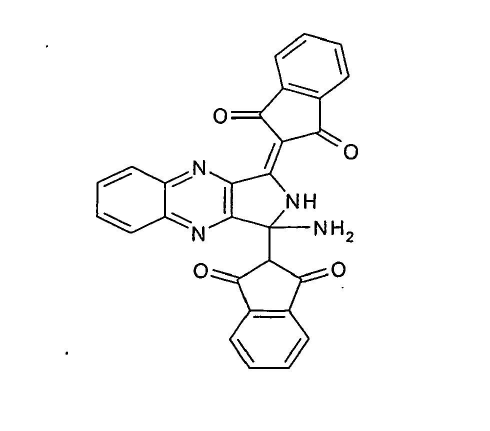 Schmelzpunkt: >300°C
MS (m/e): 473 [M+H]⁺, 495 [M+Na]⁺

### Beispiel G: 1-Amino-1-(2,4-dioxo-10H-benzo[4,5]imidazo[1,2-a]pyrimidin-3-yl)-3-(2,4-dioxo-10H-benzo[4,5]imidazo[1,2-a]pyrimidin-3-yliden)-4,9-diazabenzo[f]isoindol

Zu einer Suspension von 10,0 g 2,3-Dicyanochinoxalin in 100 ml Methanol werden bei 10°C 1,1 g Natriummethylatlösung (30 % in Methanol) getropft und 16 Stunden bei Raumtemperatur gerührt. Die Mischung wird mit 200 ml Methanol verdünnt und nach Zugabe von 2,5 g Eisessig und 23,5 g 1 H-Benz[4,5]-imidazo[1,2-a]-pyrimidin-2,4-dion 6 Stunden am Rückfluss gerührt. Die Suspension wird nach Abkühlen auf Raumtemperatur filtriert, mit Methanol, dann Wasser gewaschen und bei 60°C getrocknet. Es werden 25,9 g (80 % d.Th.) eines hellbraunen Pulvers der folgenden Formel erhalten 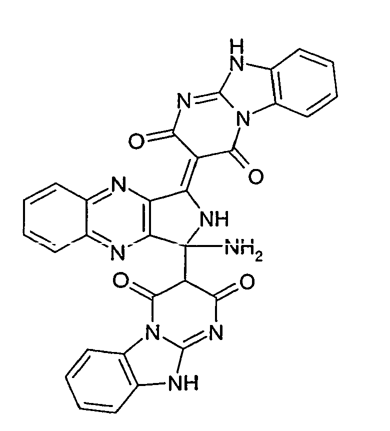 Schmelzpunkt: >300°C
MS (m/e): 583 [M+H]⁺, 605 [M+Na]⁺

### Farbmittel

### Beispiel 1: 1,3-Bis-(2,4,6-trioxo-tetrahydropyrimidin-5-yliden)-4,9-diazabenzo[f]isoindol

137 g 1-Amino-1-(2,4,6-trioxo-tetrahydropyrimidin-5-yl)-3-(2,4,6-trioxotetrahydropyrimidin-5-yliden)-4,9-diazabenzo[f]isoindol werden 30 min bei Raumtemperatur in 2060 ml Schwefelsäure (80 %) gerührt. Diese Lösung wird innerhalb von 15 min gleichmäßig unter Rühren in 4900 ml Wasser (20°C) getropft und weitere 30 min stehen gelassen. Die Suspension wird filtriert, mit Wasser neutral gewaschen und bei 60°C getrocknet. Es werden 129 g (98 % d.Th.) einer gelben Verbindung der folgenden Formel erhalten 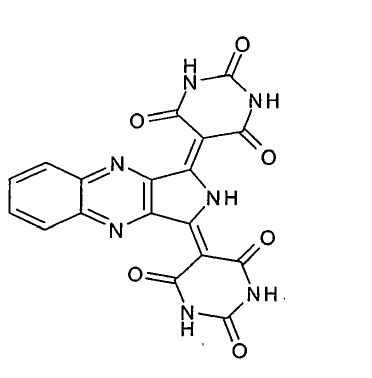 Schmelzpunkt: >300°C
MS (m/e): 420 [M+H]⁺, 442 [M+Na]⁺

### Beispiel 2: 1,3-Bis-(1,3-dimethyl-2,4,6-trioxo-tetrahydropyrimidin-5-yliden)-4,9-diazabenzo[f]isoindol

24 g 1-Amino-1-(1,3-dimethyl-2,4,6-trioxo-tetrahydropyrimidin-5-yl)-3-(1,3-dimethyl-2,4,6-trioxo-tetrahydropyrimidin-5-yliden)-4,9-diazabenzo[f]isoindol werden 30 min bei Raumtemperatur in 480 ml konz. Schwefelsäure gerührt. Diese Lösung wird innerhalb von 15 min gleichmäßig unter Rühren in 4800 ml Wasser getropft und weitere 30 min stehen gelassen. Die Suspension wird filtriert, mit Wasser neutral gewaschen und bei 60°C getrocknet. Es werden 16,7 g (72 % d.Th.) einer gelben Verbindung der folgenden Formel erhalten 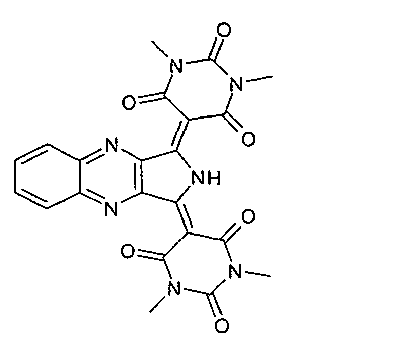 Schmelzpunkt: >300°C
MS (m/e): 476 [M+H]⁺, 498 [M+Na]⁺

### Beispiel 3: 1,3-Bis-(1,3-dioxoindan-2-yliden)-4,9-diazabenzo[f]isoindol

5,0 g 1-Amino-1-(1,3-dioxoindan-2-yl)-3-(1,3-dioxoindan-2-yliden)-4,9-diazabenzo-[f]isoindol werden 30 min bei Raumtemperatur in 75 ml konz. Schwefelsäure gerührt. Diese Lösung wird innerhalb von 15 min gleichmäßig unter Rühren in 180 ml Wasser getropft und weitere 30 min stehen gelassen. Die Suspension wird filtriert, mit Wasser neutral gewaschen und bei 60°C getrocknet. Es werden 4,49 g (93 % d.Th.) einer rotstichig gelben Verbindung der folgenden Formel erhalten 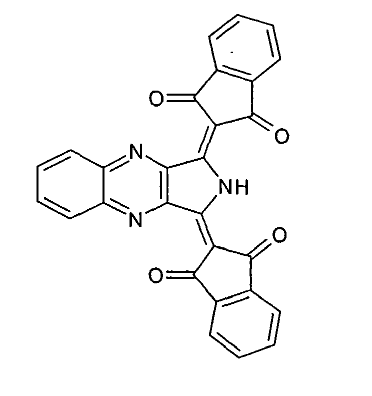 Schmelzpunkt: >300°C
MS (m/e): 476 [M+H]⁺, 498 [M+Na]⁺

### Beispiel 4: 1,3-Bis-(2,4-dioxo-1 0H-benzo[4,5]imidazo[1,2-a]pyrimidin-3-yliden)-4,9-diazabenzo[f]isoindol

5,0 g 1-Amino-1-(2,4-dioxo-10H-benzo[4,5]imidazo[1,2-a]pyrimidin-3-yl)-3-(2,4-dioxo-10H-benzo[4,5]imidazo[1,2-a]pyrimidin-3-yliden)-4,9-diazabenzo[f]isoindol werden 30 min bei Raumtemperatur in 75 ml konz. Schwefelsäure gerührt. Diese Lösung wird innerhalb von 15 min gleichmäßig unter Rühren in 180 ml Wasser getropft und weitere 30 min stehen gelassen. Die Suspension wird filtriert, mit Wasser neutral gewaschen und bei 60°C getrocknet. Es werden 4,14 g (85 % d.Th.) einer gelbstichig braunen Verbindung der folgenden Formel erhalten 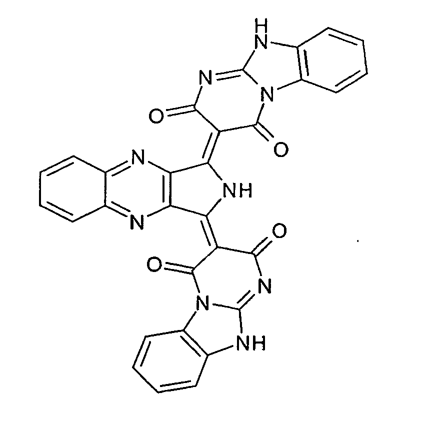 Schmelzpunkt: >300°C
MS (m/e): 566 [M+H]⁺, 588 [M+Na]⁺

### Anwendungsbeispiele

Zur Beurteilung der Eigenschaften der nach der Erfindung hergestellten Pigmente auf dem Lacksektor wurden aus der Vielzahl der bekannten Lacke ein aromatenhaltiger Alkydmelaminharzlack (AM) auf Basis eines mittelöligen Alkydharzes und eines butanolveretherten Melaminharzes, sowie ein aromatenfreier lufttrocknender Alkydharzlack (LA) auf Basis eines langöligen Sojaalkydharzes ausgewählt.

Anwendungsbeispiel 1:
Eine Applikation des Pigments aus Beispiel 1 in AM-Lack ergibt farbstarke, im Vollton und in der Aufhellung grünstichig gelbe Lackierungen.

Anwendungsbeispiel 2:
Eine Applikation des Pigments aus Beispiel 2 in LA-Lack ergibt farbstarke, im Vollton und in der Aufhellung rotstichig gelbe Lackierungen.

Anwendungsbeispiel 3:
Eine Applikation des Pigments aus Beispiel 3 in LA-Lack ergibt farbstarke, im Vollton und in der Aufhellung rotstichig gelbe Lackierungen.

Anwendungsbeispiel 4:
Eine Applikation des Pigments aus Beispiel 4 in LA-Lack ergibt im Vollton und in der Aufhellung rotstichig gelbe Lackierungen.

## Patentansprüche

1. Verbindung der allgemeinen Formel (I) 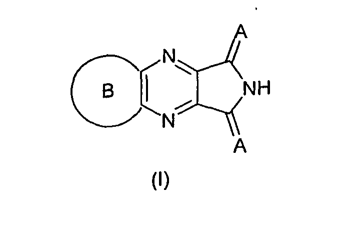 worin A eine Gruppe der allgemeinen Formel (II), (III), (IV) oder (V) ist 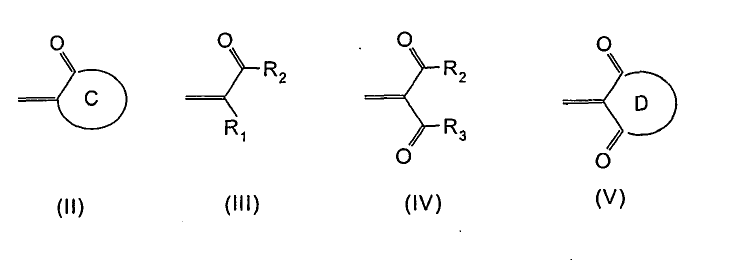 worin C und D für eine alicyclische oder heterocyclische Gruppe stehen;
R₁ für CN oder für einen 5- bis 7-gliedrigen heteroaromatischen Rest mit 1, 2 oder 3 Heteroatomen aus der Gruppe N, O und S steht,
und R₂ und R₃ unabhängig voneinander C₁-C₂₅-Alkyl, C₅-C₁₂-Cycloalkyl, C₆-C₂₄-Aryl, OH, OR₄ oder NR₄R₅ bedeuten, worin R₄ und R₅ gleich oder verschieden sind und für Wasserstoff, C₁-C₂₅-Alkyl, C₅-C₁₂-Cycloalkyl, unsubstituiertes oder durch 1, 2, 3 oder 4 Reste Halogen, R⁰, OR⁰, SR⁰, NH₂, NHR⁰, NR⁰₂, NO₂, COOH, COOR⁰, CONH₂, CONHR^{0,} CONR⁰₂, CN, SO₃H, SO₂(OR⁰), SO₂R⁰, SO₂NHR⁰, SO₂NR⁰₂ oder durch einen 5- bis 7-gliedrigen heteroaromatischen Rest mit 1, 2 oder 3 Heteroatomen aus der Gruppe N, O und S substituiertes C₆-C₂₄-Aryl oder einen 5- bis 7-gliedrigen heteroaromatischen Rest mit 1, 2 oder 3 Heteroatomen aus der Gruppe N, O und S stehen,
wobei R⁰ für C₁-C₁₈-Alkyl oder C₆-C₂₄-Aryl steht;
und B unsubstituiertes oder 1- bis 4-fach substituiertes ortho-C₆-C₁₈-Arylen bedeutet.

2. Verbindung nach Anspruch 1, worin A einen zweiwertigen alicyclischen oder heterocyclischen Rest der Formeln (a) bis (g) bedeutet, 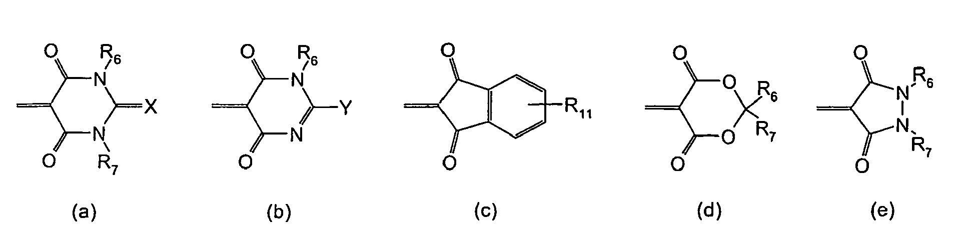 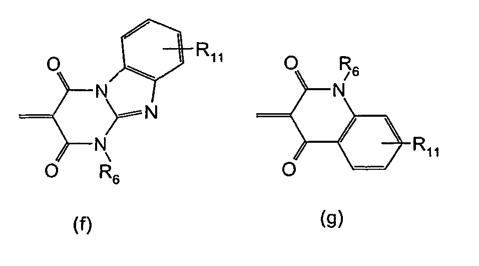 wobei R₆ und R₇ unabhängig voneinander für Wasserstoff, C₁-C₂₅-Alkyl, C₅-C₁₂-Cycloalkyl, C₆-C₂₄-Aryl, C₁-C₂₅-Alkyl-(C₆-C₁₀-aryl), einen 5- bis 7-gliedrigen heteroaromatischen Rest mit 1, 2 oder 3 Heteroatomen aus der Gruppe N, O und S, -(CH₂)ₙ-COR₈ oder -(CH₂)ₘ-OR₉, stehen, worin R₈ für Hydroxy, Amino, unsubstituiertes oder ein- oder mehrfach mit Hydroxy oder Amino substituiertes C₁-C₂₅-Alkoxy, C₁-C₂₅-Alkylamino, Di-(C₁-C₂₅-alkyl)-amino, C₁-C₂₅-Alkyl-(C₆-C₁₀-aryl)-amino, (C₆-C₂₄-Aryl)-amino, Di-(C₆-C₂₄-Aryl)-amino, C₁-C₂₅-Alkyl-(C₆-C₁₀-aryl)-amino, oder C₂-C₂₄-Alkenyloxy steht, und R₉ für Wasserstoff oder -CO-(C₁-C₂₅-Alkyl) steht, und n und m unabhängig voneinander für eine ganze Zahl von 0 bis 6 stehen, und worin in R₆, R_{7,} R₈ und R₉ eine C-C-Einheit auch durch eine Ethereinheit C-O-C ersetzt sein kann,
X für =O, =S oder =NR₁₀ steht, worin R₁₀ eine der Bedeutungen von R₆ hat,
Y für Wasserstoff, R₇, OR₇, SR₇, NHCN oder NR₇R₁₀ steht,
und R₁₁ Wasserstoff, Halogen, CN, R₇, OR₇, SR₇, NR₇R₁₀, NO₂, SO₂(OR₇), SO₂R₇, SO₂NHR₇, SO₂N(R₇)₂ oder PO₂(OR₇) bedeutet.

3. Verbindung nach Anspruch 1 oder 2, worin R₆ und R₇ Wasserstoff, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, Hydroxycarbonyl-C₀-C₆-alkyl, C₁-C₁₈-Alkoxycarbonyl-C₀-C₆-alkyl, Aminocarbonyl-C₀-C₆-alkyl, C₁-C₁₈-Alkylaminocarbonyl-C₀-C₆-alkyl, C₆-C₁₀-Arylaminocarbonyl-C₀-C₆-alkyl, Di(C₁-C₁₈-alkyl)-aminocarbonyl-C₀-C₆-alkyl, C₁-C₁₈-Alkyl-C₆-C₁₀-arylaminocarbonyl-C₀-C₆-alkyl oder Di(C₆-C₁₀-aryl)-aminocarbonyl-C₀-C₆-alkyl bedeuten.

4. Verbindung nach Anspruch 2, worin R₈ Hydroxy, C₁-C₁₈-Alkoxy, C₁-C₁₈-Alkylamino, Di(C₁-C₁₈-alkyl)-amino, Benzylamino, C₆-C₁₀-Arylamino, Di(C₆-C₁₀-aryl)-amino oder (C₂-C₁₈)-Alkenyloxy bedeutet.

5. Verbindung nach Anspruch 2, worin R₁₁ Wasserstoff, Cl, Br, C₁-C₁₈-Alkyl, C₅-C₆-Cycloalkyl, Benzyl, C₆-C₁₀-Aryl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈-Alkylthio, C₆-C₁₀-Arylthio, C₁-C₁₈-Alkylamino, C₆-C₁₀-Arylamino, Di(C₁-C₁₈-alkyl)-amino, C₁-C₁₈-Alkyl-(C₆-C₁₀-aryl)-amino, Di(C₆-C₁₀-aryl)-amino, SO₃H, C₁-C₁₈-Alkoxysulfonyl, C₁-C₁₈-Alkylsulfonyl, C₁-C₁₈-alkylaminosulfonyl oder Di(C₁-C₁₈-alkyl)-aminosulfonyl bedeutet.

6. Verfahren zur Herstellung einer Verbindung nach einem oder mehreren der Ansprüche 1 bis 5, durch Umsetzung eines 2,3-Dicyanochinoxalins der Formel (XIV) 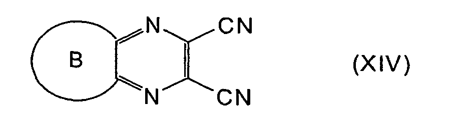 mit insgesamt mindestens 2 Equivalenten Ammoniak und/oder Alkoholaten MOR₁₂, worin M Natrium oder Kalium bedeutet, zu di- oder
monoiminosubstituierten Diazabenzoisoindolen der Formeln (VI), (VII) oder (VIII), 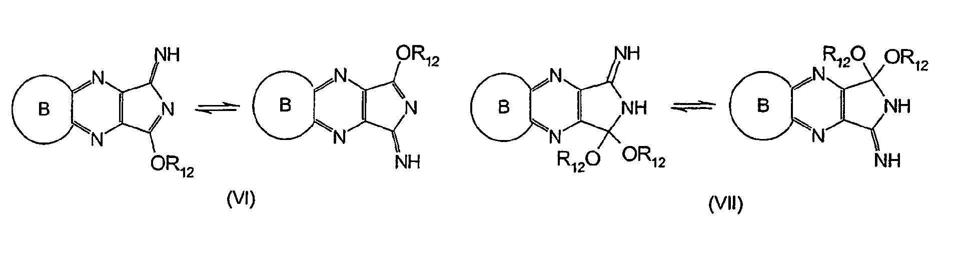 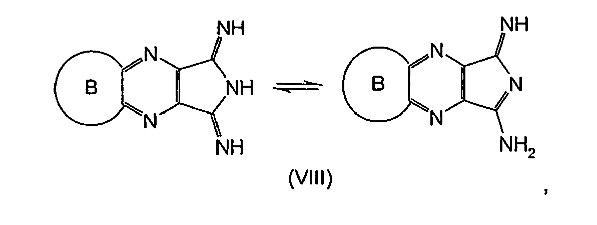 worin R₁₂ für C₁-C₁₈-Alkyl oder -(CH₂)ₘ-OH steht und m eine ganze Zahl im Bereich von 1 bis 6 bedeutet, und eine C-C- Einheit auch durch eine Ethereinheit C-O-C ersetzt werden kann,
in einem Lösemittel oder Lösemittelgemisch unter basischen bis neutralen Bedingungen bei einer Temperatur von -20 bis 120°C, die anschließend in einem Lösungsmittel oder Lösungsmittelgemisch unter neutralen bis sauren Bedingungen, mit mindestens 2 Equivalenten einer Verbindung der Formeln (IX), (X), (XI) oder (XII) 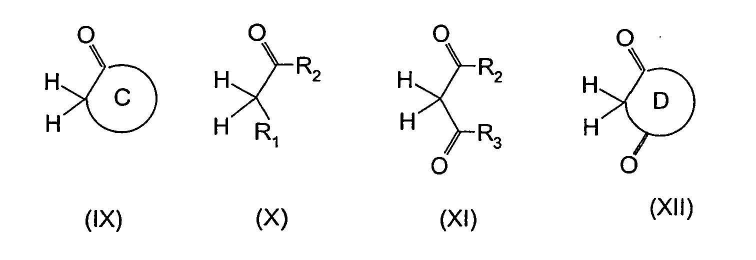 zu einem weiteren Zwischenprodukt der allgemeinen Formel (XIIIa) oder (XIIIb) 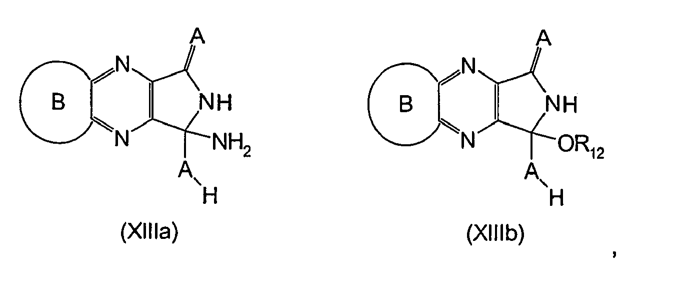 umgesetzt werden,
von dem anschließend ein Mol Ammoniak oder HOR₁₂ eliminiert wird.

7. Verfahren nach Anspruch 6, **dadurch gekennzeichnet, dass** das 2,3-Dicyanochinoxalin durch Umsetzung von 2,3-Dichlorchinoxalinen der Formel (XV) 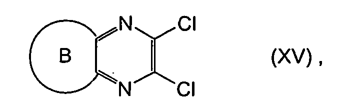 mit einem Cyanid eines Hauptgruppen- oder Übergangsgruppenmetalls in einem organischen Lösungsmittel in Gegenwart eines Phasentransferkatalysators bei erhöhten Temperaturen hergestellt wird.

8. Verbindung der allgemeinen Formel (XIIIa), 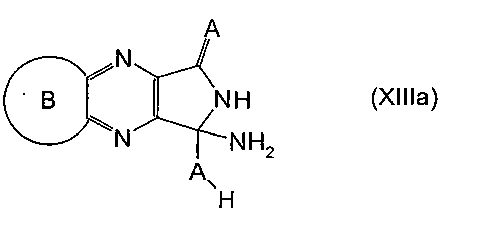 worin A und B die in einem oder mehreren der Ansprüche 1 bis 5 genannten Bedeutungen haben.

9. Verwendung einer Verbindung gemäß einem oder mehreren der Ansprüche 1 bis 5 zum Einfärben oder Pigmentieren von organischen oder anorganischen, hoch- oder niedermolekularen Materialien.

10. Verwendung nach Anspruch 9 als Farbmittel in Anstrichfarben auf öliger oder wässriger Grundlage, in Lacken, Tarnfarben, zum Spinnfärben, zum Massefärben oder Pigmentieren von Kunststoffen, in Druckfarben, in der Papiermassefärbung, für Saatgut, zur Herstellung von Tinten, Ink-Jet Tinten auf wässriger oder nichtwässriger Basis, Mikroemulsionstinten und Tinten, die nach dem Hot-melt Verfahren arbeiten.

11. Verwendung nach Anspruch 9 als Farbmittel für elektrophotographische Toner und Entwickler, für Farbfilter, für elektronische Tinten, für Pulverlacke, sowie in optischen Schichten für die optische Datenspeicherung.

12. Zusammensetzung, enthaltend ein organisches oder anorganisches, hochmolekulares oder niedermolekulares Material und eine Verbindung nach einem oder mehreren der Ansprüche 1 bis 5 in einer Menge von 0,005 bis 70 Gew.-%, bezogen auf das organische oder anorganische Material.

## Claims

1. A compound of the general formula (I) 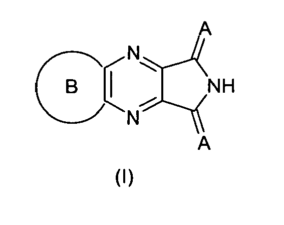 in which A is a group of the general formula (II), (III), (IV) or (V) 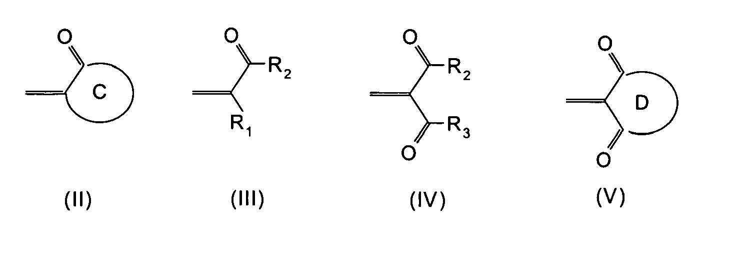 in which C and D are an alicyclic or heterocyclic group;
R₁ is CN or is a 5- to 7-membered heteroaromatic radical having 1, 2 or 3 heteroatoms from the group N, O, and S,
and R₂ and R₃ independently of one another are C₁-C₂₅ alkyl , C₅-C₁₂ cycloalkyl, C₆-C₂₄ aryl, OH, OR₄ or NR₄R₅, in which R₄ and R₅ are identical or different and are hydrogen, C₁-C₂₅ alkyl, C₅-C₁₂ cycloalkyl, C₆-C₂₄ aryl which is unsubstituted or substituted by 1, 2, 3 or 4 radicals halogen, R⁰, OR⁰, SR⁰, NH₂, NHR⁰, NR⁰₂, NO₂, COOH, COOR⁰, CONH₂, CONHR⁰, CONR⁰₂, CN, SO₃H , SO₂(OR⁰), SO₂R⁰, SO₂NHR⁰, SO₂NR⁰₂ or by a 5- to 7-membered heteroaromatic radical having 1, 2 or 3 heteroatoms from the group N, O, and S, or are a 5- to 7-membered heteroaromatic radical having 1, 2 or 3 heteroatoms from the group N, O, and S,
R⁰ being C₁-C₁₈ alkyl or C₆-C₂₄ aryl ;
and B is unsubstituted or mono- to tetrasubstituted ortho-C₆-C₁₈ arylene.

2. A compound as claimed in claim 1, in which A is a divalent alicyclic or heterocyclic radical of the formulae (a) to (g) 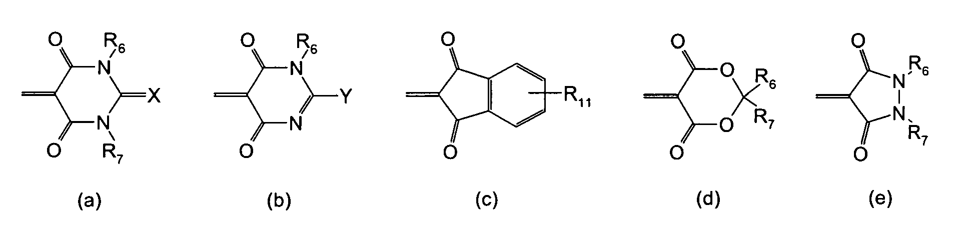 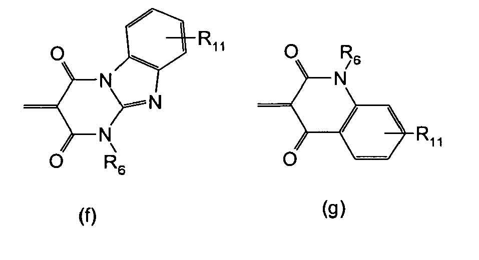 where R₆ and R₇ independently of one another are hydrogen, C₁-C₂₅ alkyl , C₅-C₁₂ cycloalkyl , C₆-C₂₄ aryl, C₁-C₂₅ alkyl (C₆-C₁₀ aryl), a 5- to 7-membered heteroaromatic radical having 1, 2 or 3 heteroatoms from the group N, O, and S, -(CH₂)ₙ-COR₈ or -(CH₂)ₘ-OR₉, in which R₈ is hydroxyl, amino, unsubstituted or mono- or polyhydroxyl-substituted or mono- or polyamino-substituted C₁-C₂₅ alkoxy, C₁-C₂₅ alkylamino, di (C₁-C₂₅ alkyl) amino, C₁-C₂₅ alkyl (C₆-C₁₀ aryl) amino, (C₆-C₂₄ aryl) amino, di(C₆-C₂₄ aryl) amino, C₁-C₂₅ alkyl(C₆-C₁₀ aryl)amino, or C₂-C₂₄ alkenyloxy,
and R₉ is hydrogen or -CO-(C₁-C₂₅ alkyl), and n and m independently of one another are an integer from 0 to 6, and in which in R₆, R₇, R₈, and R₉ it is also possible for a C-C unit to be replaced by an ether unit C-O-C, X is =O, =S or =NR₁₀, in which R₁₀ has one of the definitions of R₆,
Y is hydrogen, R₇, OR₇, SR₇, NHCN or NR₇R₁₀,
and R₁₁ is hydrogen, halogen, CN, R₇, OR₇, SR₇, NR₇R₁₀, NO₂, SO₂(OR₇), SO₂R₇, SO₂NHR₇, SO₂N(R₇)₂ or PO₂(OR₇).

3. A compound as claimed in claim 1 or 2, in which R₆ and R₇ are hydrogen, C₁-C₁₈ alkyl, C₅-C₆ cycloalkyl, C₆-C₁₀ aryl, benzyl, pyridyl, pyrryl, thienyl, imidazolyl, oxazolyl, thiazolyl, pyrimidyl, hydroxycarbonyl-C₀-C₆ alkyl, C₁-C₁₈ alkoxycarbonyl-C₀-C₆ alkyl, aminocarbonyl-C₀-C₆ alkyl, C₁-C₁₈ alkylaminocarbonyl-C₀-C₆ alkyl, C₆-C₁₀ arylaminocarbonyl-C₀-C₆ alkyl, di (C₁-C₁₈ alkyl) aminocarbonyl-C₀-C₆ alkyl, C₁-C₁₈ alkyl-C₆-C₁₀ arylaminocarbonyl-C₀-C₆ alkyl or di (C₆-C₁₀ aryl)aminocarbonyl-C₀-C₆ alkyl.

4. A compound as claimed in claim 2, in which R₈ is hydroxyl, C₁-C₁₈ alkoxy, C₁-C₁₈ alkylamino, di (C₁-C₁₈ alkyl)amino, benzylamino, C₆-C₁₀ arylamino, di (C₆-C₁₀ aryl)amino or (C₂-C₁₈) alkenyloxy.

5. A compound as claimed in claim 2, in which R₁₁ is hydrogen, Cl, Br, C₁-C₁₈ alkyl, C₅-C₆ cycloalkyl, benzyl, C₆-C₁₀ aryl, pyridyl, pyrryl, thienyl, imidazolyl, oxazolyl, thiazolyl, pyrimidyl, C₁-C₁₈ alkoxy, C₆-C₁₀ aryloxy, C₁-C₁₈ alkylthio, C₆-C₁₀ arylthio, C₁-C₁₈ alkylamino, C₆-C₁₀ arylamino, di(C₁-C₁₈ alkyl)amino, C₁-C₁₈ alkyl (C₆-C₁₀ aryl) amino, di (C₆-C₁₀ aryl) amino, SO₃H, C₁-C₁₈ alkoxysulfonyl, C₁-C₁₈ alkylsulfonyl, C₁-C₁₈ alkylaminosulfonyl or di (C₁-C₁₈ alkyl)aminosulfonyl.

6. A process for preparing a compound as claimed in one or more of claims 1 to 5, by reacting a 2,3-dicyanoquinoxaline of the formula (XIV) 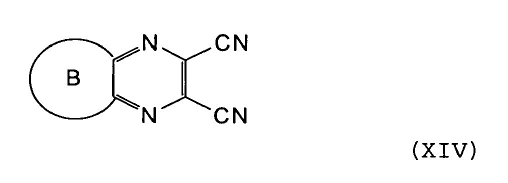 with a total of at least 2 equivalents of ammonia and/or alkoxides MOR₁₂, in which M is sodium or potassium, to give di- or monoimino-substituted diazabenzoisoindoles of the formulae (VI), (VII) or (VIII) 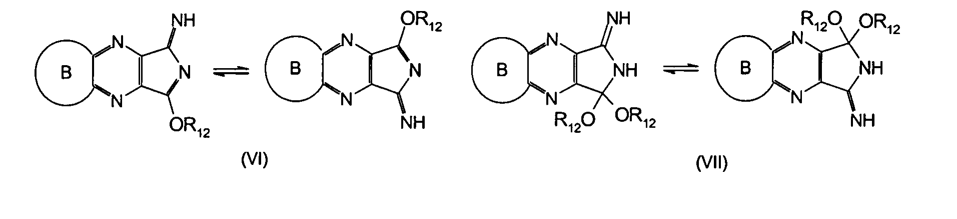 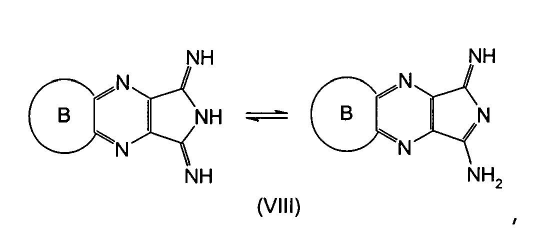 in which R₁₂ is C₁-C₁₈ alkyl or -(CH₂)ₘ-OH and m is an integer in the range from 1 to 6, and it is also possible for a C-C unit to be replaced by an ether unit C-O-C, in a solvent or solvent mixture under basic to neutral conditions at a temperature of -20 to 120°C, which are subsequently reacted, in a solvent or solvent mixture under neutral to acidic conditions, with at least 2 equivalents of a compound of the formulae (IX), (X), (XI) or (XII) 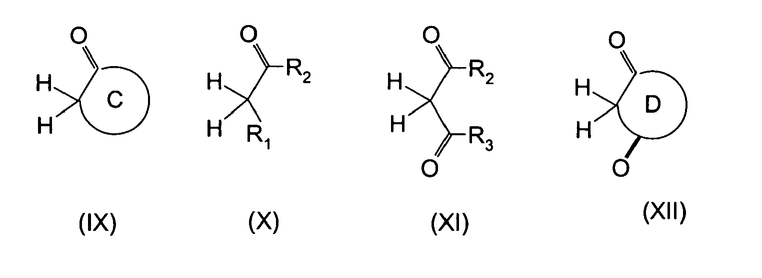 to give a further intermediate of the general formula (XIIIa) or (XIIIb) 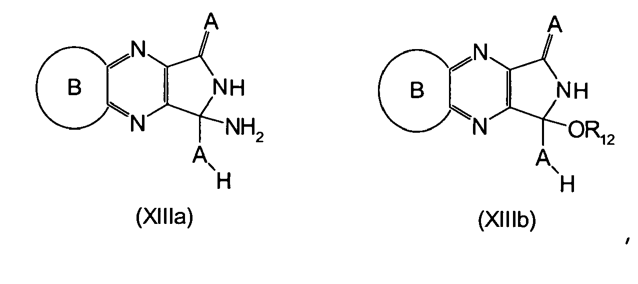 from which subsequently one mole of ammonia or HOR₁₂ is eliminated

7. The process as claimed in claim 6, wherein the 2,3-dicyanoquinoxaline is prepared by reacting 2,3-dichloroquinoxalines of the formula (XV) 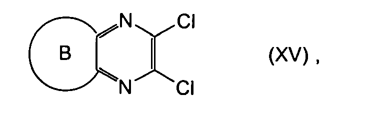 with a cyanide of a main-group or transition-group metal in an organic solvent in the presence of a phase-transfer catalyst at elevated temperatures.

8. A compound of the general formula (XIIIa), 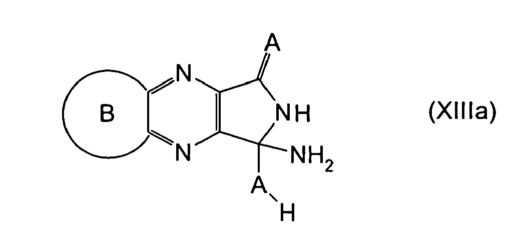 in which A and B are as defined in one or more of claims 1 to 5.

9. The use of a compound as claimed in one or more of claims 1 to 5 for dyeing or pigmenting organic or inorganic materials of high or low molecular weight.

10. The use as claimed in claim 9 as colorants in oilbased or water-based paints, in coating materials, camouflage paints, for spin coloring, for the mass coloring or pigmenting of plastics, in printing inks, in the mass coloring of paper, for seed, for preparing inks, water-based or non-water-based ink-jet inks, microemulsion inks, and inks which operate in accordance with the hot-melt process.

11. The use as claimed in claim 9 as colorants for electrophotographic toners and developers, for color filters, for electronic inks, for powder coating materials, and in optical layers for optical data storage.

12. A composition comprising an organic or inorganic, high or low molecular weight material and a compound as claimed in one or more of claims 1 to 5 in an amount of 0.005% to 70% by weight, based on the organic or inorganic material.

## Revendications

1. Composé de formule générale (I) 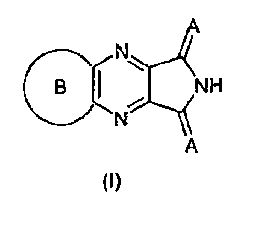 dans laquelle A représente un groupement de formule générale (II), (III), (IV) ou (V) 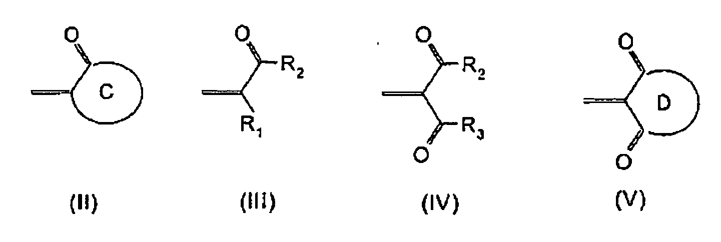 dans lesquelles C et D représentent un groupement alicyclique ou hétérocyclique ;
R₁ représente CN ou un radical hétéroaromatique ayant de 5 à 7 éléments avec 1, 2 ou 3 hétéroatomes choisis dans le groupe N, O et S,
et R₂ et R₃ représentent indépendamment l'un de l'autre un alkyle en C₁-C₂₅, un cycloalkyle en C₅-C₁₂, un aryle en C₆-C₂₄, OH, OR₄ ou NR₄R₅, R₄ et R₅ étant identiques ou différents et représentant l'hydrogène, un alkyle en C₁-C₂₅, un cycloalkyle en C₅-C₁₂, un aryle en C₆-C₂₄ non substitué ou substitué par 1, 2, 3 ou 4 radicaux halogène, R⁰ , OR⁰ , SR⁰ , NH₂, NHR⁰, NR⁰₂, NO₂, COOH, COOR⁰ , CONH₂, CONHR⁰ , CONR⁰₂, CN, SO₃H, SO₂(OR⁰), SO₂R⁰, SO₂NHR⁰, SO₂NR⁰₂ ou par un radical hétéroaromatique ayant de 5 à 7 éléments avec 1, 2 ou 3 hétéroatomes choisis dans le groupe N, O et S ou un radical hétéroaromatique ayant de 5 à 7 éléments avec 1, 2 ou 3 hétéroatomes choisis dans le groupe N, O et S,
R° représentant un alkyle en C₁-C₁₈ ou un aryle en C₆-C₂₄ ;
et B représente un ortho-C₆-C₁₈-arylène non substitué ou substitué 1 à 4 fois.

2. Composé selon la revendication 1, dans lequel A représente un radical bivalent alicyclique ou hétérocyclique selon des formules (a) à (g), 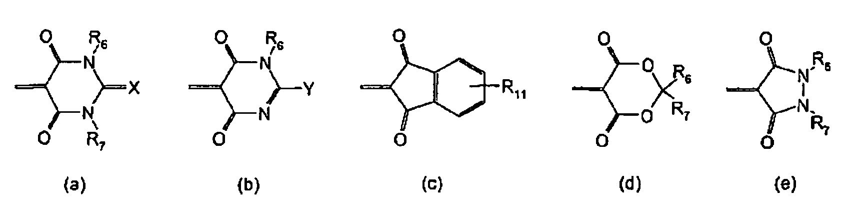 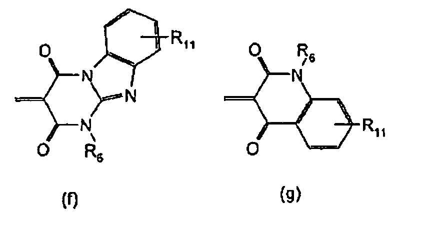 dans lesquelles R₆ et R₇ représentent indépendamment l'un de l'autre l'hydrogène, un alkyle en C₁-C₂₅, un cycloalkyle en C₅-C₁₂, un aryle en C₆-C₂₄, un C₁-C₂₅-alkyl-(C₆-C₁₀-aryl), un radical hétéroaromatique ayant de 5 à 7 éléments avec 1, 2 ou 3 hétéroatomes choisis dans le groupe N, O et S, (CH₂)ₙ-COR₈ ou (CH₂)ₘ-OR₉, R₈ représentant un hydroxy, un amino, un alcoxy en C₁-C₂₅ non substitué ou substitué une ou plusieurs fois par un hydroxy ou un amino, un alkylamino en C₁-C₂₅, un di- (C₁-C₂₅-alkyl)-amino, un C₁-C₂₅-alkyl- (C₆-C₁₀-aryl) -amino, un (C₆-C₂₄-aryl)-amino, un di-(C₆-C₂₄-aryl)-amino, un C₁-C₂₅-alkyl-(C₆-C₁₀-aryl)-amino ou un alcényloxy en C₂-C₂₄, et R₉ représentant l'hydrogène ou un -CO-(C₁-C₂₅-alkyl), et n et m représentant indépendamment l'un de l'autre un nombre entier de 0 à 6, et, dans R₆, R₇, R₈ et R₉, une unité C-C pouvant être remplacée par une unité éther C-O-C,
X représente =O, =S ou =NR₁₀, R₁₀ ayant une des significations de R₆,
Y représente l'hydrogène, R₇, OR₇, SR₇, NHCN ou NR₇R₁₀, et R₁₁ représente l'hydrogène, un halogène, CN, R₇, OR₇, SR₇, NR₇R₁₀, NO₂, SO₂(OR₇), SO₂R₇, SO₂NHR₇, SO₂N (R₇)₂ ou PO₂(OR₇).

3. Composé selon la revendication 1 ou 2, dans lequel R₆ et R₇ représentent l'hydrogène, un alkyle en C₁-C₁₈, un cycloalkyle en C₅-C₆, un aryle en C₆-C₁₀, un benzyle, un pyridyle, un pyrryle, un thiényle, un imidazolyle, un oxazolyle, un thiazolyle, un pyrimidyle, un hydroxycarbonyle-C₀-C₆-alkyle, un C₁-C₁₈-alcoxycarbonyl-C₀-C₆-alkyle, un aminocarbonyl-C₀-C₆-alkyle, un C₁-C₁₈-alkylaminocarbonyl-C₀-C₆-alkyle, un C₆-C₁₀-arylaminocarbonyle-C₀-C₆-alkyle, un di(C₁-C₁₈-alkyl) - aminocarbonyl-C₀-C₆-alkyle, un C₁-C₁₈-alkyl-C₆-C₁₀-arylaminocarbonyl-C₀-C₆-alkyle ou un di (C₆-C₁₀-aryl) - aminocarbonyl-C₀-C₆-alkyle.

4. Composé selon la revendication 2, dans lequel R₈ représente un hydroxy, un alcoxy en C₁-C₁₈, un alkylamino en C₁-C₁₈, un di (C₁-C₁₈-alkyl) -amino, un benzylamino, un arylamino en C₆-C₁₀, un di(C₆-C₁₀-aryl)-amino ou un alcényloxy en C₂-C₁₈.

5. Composé selon la revendication 2, dans lequel R₁₁ représente l'hydrogène, Cl, Br, un alkyle en C₁-C₁₈, un cycloalkyle en C₅-C₆, un benzyle, un aryle en C₆-C₁₀, un pyridyle, un pyrryle, un thiényle, un imidazolyle, un oxazolyle, un thiazolyle, un pyrimidyle, un alcoxy en C₁-C₁₈, un aryloxy en C₆-C₁₀, un alkylthio en C₁-C₁₈, un arylthio en C₆-C₁₀, un alkylamino en C₁-C₁₈, un arylamino en C₆-C₁₀, un di (C₁-C₁₈-alkyl) -amino, un C₁-C₁₈-alkyl-(C₆-C₁₀-aryl)-amino, un di (C₆-C₁₀-aryl) -amino, SO₃H, un alcoxysulfonyle en C₁-C₁₈, un alkylsulfonyle en C₁-C₁₈, un alkylaminosulfonyle en C₁-C₁₈ ou un di (C₁-C₁₈-alkyl)-aminosulfonyle.

6. Procédé de fabrication d'un composé selon une ou plusieurs des revendications 1 à 5, par réaction d'une 2,3-dicyanoquinoxaline de formule (XIV) 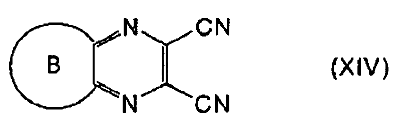 avec au total au moins 2 équivalents d'ammoniac et/ou d'alcoolates MOR₁₂, M représentant l'azote ou le potassium, pour former des diazabenzoisoindoles di- ou monoiminosubstitués de formule (VI), (VII) ou (VIII), 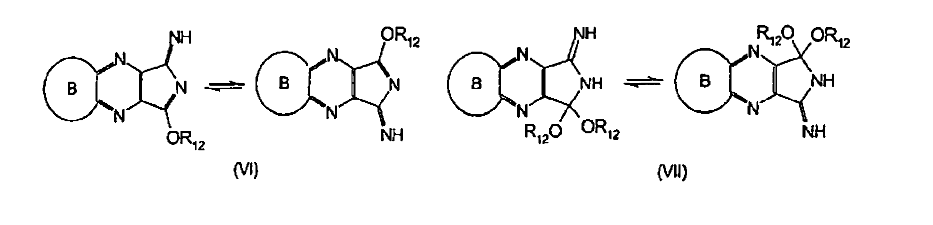 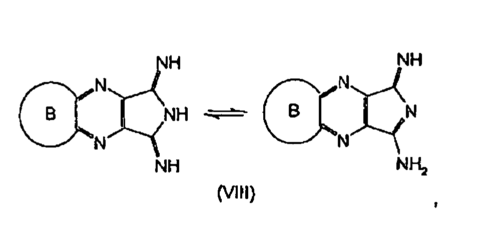 dans lesquelles R₁₂ représente un alkyle en C₁-C₁₈ ou un -(CH₂)ₘ-OH et m représente un nombre entier de 1 à 6, et une unité C-C pouvant être remplacée par une unité éther C-O-C,
dans un solvant ou un mélange de solvants en conditions basiques à neutres à une température de -20 à 120 °C, les produits étant ensuite mis en réaction dans un solvant ou un mélange de solvants en conditions neutres à acides, avec au moins 2 équivalents d'un composé de formule (IX), (X), (XI) ou (XII) 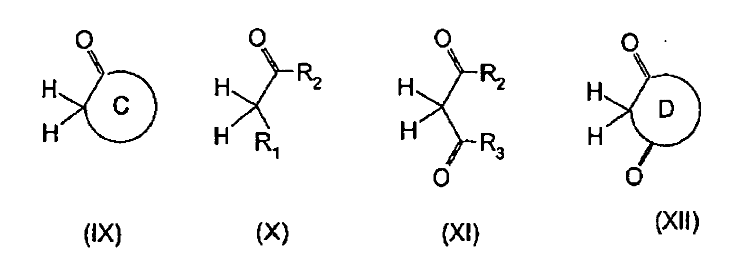 pour former un autre produit intermédiaire de formule générale (XIIIa) ou (XIIIb) 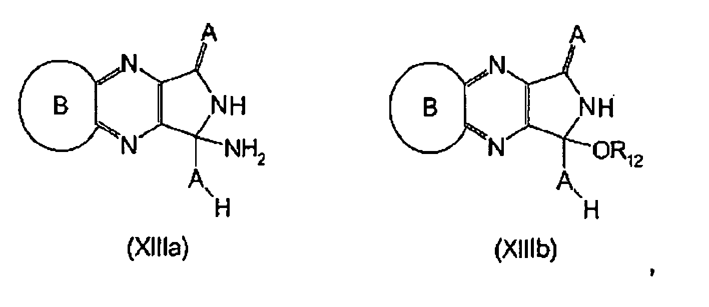 à partir duquel une mole d'ammoniac ou de HOR₁₂ est ensuite éliminée.

7. Procédé selon la revendication 6, **caractérisé en ce que** la 2,3-dicyanoquinoxaline est obtenue par réaction de la 2,3-dichloroquinoxaline de formule (XV) 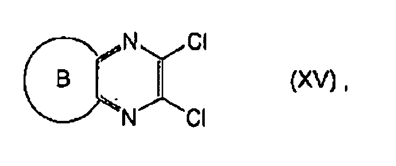 avec un cyanure d'un métal d'un groupe principal ou d'un groupe de transition dans un solvant organique en présence d'un catalyseur de transfert de phase à température élevée.

8. Composé de formule générale (XIIIa), 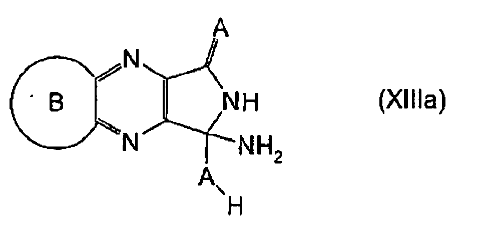 dans laquelle A et B ont les significations données dans une ou plusieurs des revendications 1 à 5.

9. Utilisation d'un composé selon une ou plusieurs des revendications 1 à 5 pour la coloration ou la pigmentation de matériaux organiques ou inorganiques, de masse moléculaire élevée ou faible.

10. Utilisation selon la revendication 9 en tant que colorant dans des peintres à base huileuse ou aqueuse, dans des laques, des peintures de camouflage, pour la teinture en solution, pour la coloration en masse ou la pigmentation de plastiques, dans des encres d'impression, pour la coloration en masse du papier, pour les semences, pour la fabrication d'encres, d'encres pour impression jet d'encre à base aqueuse ou non aqueuse, d'encres à microémulsion et d'encres fonctionnnant par le procédé d'enduction sous fusion.

11. Utilisation selon la revendication 9 en tant que colorant pour toner électrophotographique et révélateur, pour filtres colorés, pour encres électroniques, pour laques en poudre, ainsi que dans des couches optiques pour la manipulation optique de données.

12. Composition, contenant un matériau organique ou inorganiques, de masse moléculaire élevée ou faible, et un composé selon une ou plusieurs des revendications 1 à 5 en une quantité de 0,005 à 70 % en poids par rapport au matériau organique ou inorganique.
